(19) Europäisches Patentamt
European Patent Office
Office européen des brevets

(11) Veröffentlichungsnummer : **0 050 376**
**B1**

(12) # EUROPÄISCHE PATENTSCHRIFT

(45) Veröffentlichungstag der Patentschrift : **07.12.83**

(51) Int. Cl.$^3$ : **C 07 D487/04**// C07D231/38, C07D231/40 ,(C07D487/04, 243/00, 231/00)

(21) Anmeldenummer : **81110770.5**

(22) Anmeldetag : **07.08.80**

(60) Veröffentlichungsnummer der früheren Anmeldung nach Art. 76 EPÜ : **0024038**

(54) **5,6,7,8-Tetrahydropyrazole(3,4-b) (1,5)diazepin-1H, 4H-5,7-dione und Verfahren zu ihrer Darstellung.**

(30) Priorität : **14.08.79 DE 2932835**

(43) Veröffentlichungstag der Anmeldung : **28.04.82 Patentblatt 82/17**

(45) Bekanntmachung des Hinweises auf die Patenterteilung : **07.12.83 Patentblatt 83/49**

(84) Benannte Vertragsstaaten : **AT BE CH DE FR GB IT LI NL SE**

(56) Entgegenhaltungen : **Keine**

(73) Patentinhaber : **HOECHST AKTIENGESELLSCHAFT**
**Postfach 80 03 20**
**D-6230 Frankfurt am Main 80 (DE)**

(72) Erfinder : **Rackur, Gerhard, Dr.**
**Gundelhardtstrasse 2**
**D-6233 Kelkheim (Taunus) (DE)**
Erfinder : **Hoffmann, Irmgard, Dr.**
**Oranienstrasse 1**
**D-6232 Bad Soden am Taunus (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 050 376 B1

5,6,7,8-Tetrahydropyrazolo(3,4-b) (1,5)diazepin-1H, 4H-5,7-dione und Verfahren zu ihrer Darstellung

Die Erfindung betrifft 5,6,7,8-Tetrahydropyrazolo(3,4-b) (1,5)diazepin-1H, 4H-5,7-dione der allgemeinen Formel (I)

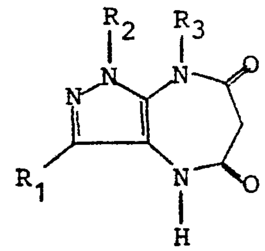

(I)

worin $R_1$ und $R_2$ gleich oder verschieden sind und Wasserstoffatome oder Alkylgruppen mit 1-6 C-Atomen darstellen, wobei einer der Reste $R_1$ und $R_2$ jeweils auch eine Benzyl-, Trifluormethyl- oder Phenylgruppe sein kann und $R_3$ ein Wasserstoffatom, eine Alkylgruppe mit 1-6 C-Atomen, die gegebenenfalls durch eine Phenylgruppe, eine Alkoxygruppe mit 1-6 C-Atomen, eine Trifluormethyl- oder eine Dialkylaminogruppe mit 2-12 C-Atomen oder eine Cycloalkylgruppe mit 3-6 C-Atomen substituiert ist, eine Alkenyl- oder Alkinylgruppe mit 2-6 C-Atomen, eine Cycloalkylgruppe mit 3-6 C-Atomen oder eine Carbalkoxygruppe mit 2-6 C-Atomen bedeutet.

Insbesondere beinhaltet die Erfindung Verbindungen, worin $R_1$ und $R_2$ gleich oder verschieden sind und Wasserstoff, Methyl-, Ethyl-, iso-Propyl-, n-Butyl bedeuten, $R_2$ manchmal auch vorteilhaft ein Phenyl- oder Benzylrest sein kann.

Für den Rest $R_3$ kommt insbesondere in Betracht ein Wasserstoffatom, eine Methyl-, Ethyl-, Benzyl-, Methoxy-, Dimethoxy- oder Trimethoxybenzyl, Propenyl-, Propinyl-, Cyclopropylmethylgruppe, eine Methoxymethylen- sowie eine Ethoxymethylengruppierung.

Verbindungen gemäß der Erfindung sind beispielsweise : 1-Methyl-5,6,7,8-tetrahydropyrazolo(3,4-b) (1,5)diazepin-1H,4H-5,7-dion, 1,8-Dimethyl-5,6,7,8-tetrahydropyrazolo(3,4-b) (1,5)diazepin-1H,4H-5,7-dion, 8-Ethyl-1-methyl-5,6,7,8-tetrahydropyrazolo(3,4-b) (1,5)diazepin-1H,4H-5,7-dion, 8-Allyl-1-methyl-5,6,7,8-tetrahydropyrazolo(3,4-b) (1,5)diazepin-1H,4H-5,7-dion, 1-Methyl-8-(2-propinyl)-5,6,7,8-tetrahydro-(3,4-b) (1,5)diazepin-1H,4H-5,7-dion, 8-Cyclopropylmethyl-1-methyl-4-5,6,7,8-tetrahydropyrazolo(3,4-b) (1,5)diazepin-1H,4H-5,7-dion, 1-Methyl-8-(2-dimethylaminoethyl)-5,6,7,8-tetrahydropyrazolo(3,4-b) (1,5)diazepin-1H,4H-5,7-dion, 8-(2-Diethylaminoethyl)-1-methyl-5,6,7,8-tetrahydropyrazolo(3,4-b) (1,5)diazepin-1H, 4H-5,7-dion, 8-(2,2,2-Trifluorethyl)-1-methyl-5,6,7,8-tetrahydropyrazolo(3,4-b) (1,5)diazepin-1H,4H-5,7-dion, 1,3-Dimethyl-8-ethyl-5,6,7,8-tetrahydropyrazolo(3,4-b) (1,5)diazepin-1H,4H-5,7-dion, 8-Allyl-1,3-dimethyl-5,6,7,8-tetrahydropyrazolo(3,4-b) (1,5)diazepin-1H,4H,5,7-dion, 1,3-Dimethyl-8-(2-propinyl)-5,6,7,8-tetrahydropyrazolo(3,4-b) (1,5)diazepin-1H,4H,5,7-dion, 1,3-Dimethyl-8-(dimethylaminoethyl)-5,6,7,8-tetrahydropyrazolo(3,4-b) (1,5)diazepin-1H,4H-5,7-dion, 8-(2-diethylaminoethyl)-1,3-dimethyl-5,6,7,8-tetrahydropyrazolo(3,4-b) (1,5)diazepin-1H,4H-5,7-dion, 1,3-Dimethyl-8-(2,2,2-trifluorethyl)-5,6,7,8-tetrahydropyrazolo(3,4-b) (1,5)diazepin-1H,4H-5,7-dion, 1,8-Diethyl-3-methyl-5,6,7,8-tetrahydropyrazolo(3,4-b) (1,5) diazepin-1H,4H,5,7-dion, 8-Allyl-1-ethyl-3-methyl-5,6,7,8-tetrahydropyrazolo(3,4-b) (1,5)-diazepin-1H,4H-5,7-dion, 1-Ethyl-3-methyl-8-(2-propinyl)-5,6,7,8-tetrahydropyrazolo(3,4-b) (1,5)diazepin-1H,4H-5,7-dion, 8-Cyclopropylmethyl-1-ethyl-3-methyl-5,6,7,8-tetrahydropyrazolo(3,4-b) (1,5)diazepin-1H,4H-5,7-dion, 1-Ethyl-3-methyl-8-(2-dimethylaminoethyl)-5,6,7,8-tetrahydropyrazolo(3,4-b) (1,5)diazepin-1H,4H-5,7-dion, 1-Ethyl-8-(2-diethylaminoethyl)-3-methyl-5,6,7,8-tetrahydropyrazolo(3,4-b) (1,5)diazepin-1H,4H-5,7-dion, 1-Ethyl-8-(2,2,2-trifluorethyl)-3-methyl-5,6,7,8-tetrahydropyrazolo(3,4-b) (1,5)diazepin-1H,4H-5,7-dion, 3-Methyl-8-ethyl-1-phenyl-5,6,7,8-tetrahydropyrazolo(3,4-b) (1,5)diazepin-1H,4H-5,7-dion, 8-Allyl-3-methyl-1-phenyl-5,6,7,8-tetrahydropyrazolo(3,4-b) (1,5)diazepin-1H,4H-5,7-dion, 3-Methyl-1-phenyl-8-(2-propinyl)-5,6,7,8-tetrahydropyrazolo(3,4-b) (1,5)-diazepin-1H,4H-5,7-dion, 8-Cyclopropylmethyl-3-methyl-1-phenyl-5,6,7,8-tetrahydropyrazolo(3,4-b) (1,5)diazepin-1H,4H-5,7-dion, 3-Methyl-8-(2-dimethylaminoethyl)-1-phenyl-5,6,7,8-tetrahydropyrazolo(3,4-b) (1,5)diazepin-1H,4H-5,7-dion, 8-(2-Diethylaminoethyl)-3-methyl-1-phenyl-5,6,7,8-tetrahydropyrazolo(3,4-b) (1,5)diazepin-1H,4H-5,7-dion, 8-(2,2,2-Trifluorethyl)-3-methyl-1-phenyl-5,6,7,8-tetrahydropyrazolo(3,4-b) (1,5)diazepin-1H,4H-5,7-dion, 1-Benzyl-8-ethyl-3-methyl-5,6,7,8-tetrahydropyrazolo(3,4-b) (1,5)diazepin-1H,4H-5,7-dion, 1-Benzyl-3-methyl-8-(2-propinyl)-5,6,7,8-tetrahydropyrazolo(3,4-b) (1,5)diazepin-1H,4H-5,7-dion, 8-Allyl-1-benzyl-3-methyl-5,6,7,8-tetrahydropyrazolo-(3,4-b) (1,5)diazepin-1H,4H-5,7-dion, 1-Benzyl-8-cyclopropylmethyl-3-methyl-5,6,7,8-tetrahydropyrazolo-(3,4-b) (1,5)diazepin-1H,4H-5,7-dion, 1-Benzyl-3-methyl-8-(2-dimethylaminoethyl)-5,6,7,8-tetrahydropyrazolo(3,4-b) (1,5)diazepin-1H,4H-5,7-dion, 1-Benzyl-8-(2-diethylaminoethyl)-3-methyl-5,6,7,8-tetrahydropyrazolo(3,4-b) (1,5)diazepin-1H,4H-5,7-dion und 1-Benzyl-8-(2,2,2-trifluorethyl)-3-methyl-tetrahydropyrazolo(3,4-b) (1,5)diazepin-1H,4H-5,7-dion.

Die Verbindungen sind Vorstufen zu pharmakologisch wirksamen Verbindungen, die in der europäischen Patentanmeldung Nr. 24 038 näher beschrieben sind.

Gegenstand der Erfindung sind weiterhin Verfahren zu ihrer Herstellung.

Die Herstellung kann in an sich bekannter Weise dadurch erfolgen, daß man
a) eine Verbindung der allgemeinen Formel (II)

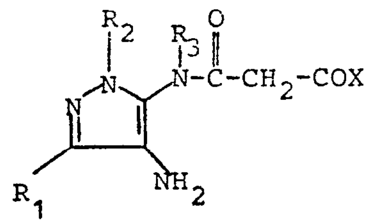

(II)

worin $R_1$, $R_2$, $R_3$ (mit Ausnahme von Wasserstoff) die oben angegebenen Bedeutungen haben und X eine Hydroxygruppe, eine Mercaptogruppe, ein Halogenatom, eine Alkoxygruppe, eine Alkylmercaptogruppe, eine Aminogruppe, eine Alkylaminogruppe, eine Dialkylaminogruppe, eine Benzoyloxygruppe, eine Aryloxygruppe, eine Acyloxygruppe oder die Gruppe $N_3$ bedeutet, zum Siebenring zyklisiert ; oder

b) eine Verbindung der allgemeinen Formel (III)

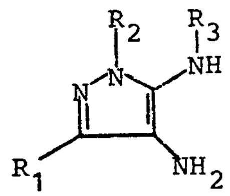

(III)

wobei $R_1$, $R_2$ und $R_3$ (mit Ausnahme von Wasserstoff) die oben angegebenen Bedeutungen haben, mit einem aktivierten Malonsäurederivat, z. B. Malonsäuredihalogenid, Malonester, Kohlensuboxyd, Meldrumsäure oder mit Malonsäure selbst zum Siebenring kondensiert ; oder

c) eine Verbindung der allgemeinen Formel (IV)

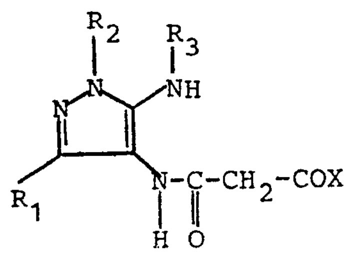

(IV)

wobei $R_1$, $R_2$, $R_3$ (mit Ausnahme von Wasserstoff) die oben angegebenen Bedeutungen haben und für X die unter a) angegebenen Gruppen in Frage kommen, mit den unter a) beschriebenen Verfahren zum Siebenring zyklisiert.

Das Verfahren a) kann mit oder ohne Lösungsmittel durch Erhitzen auf 50-250 °C, gegebenenfalls unter Zusatz eines für derartige Reaktionen üblichen Kondensationsmittels, durchgeführt werden. Als Lösungsmittel kommen z. B. in Frage : Aliphatische Alkohole (Methanol, Ethanol), Dioxan, DMF, Benzol, Toluol, Eisessig, Polyphosphorsäure, $H_2SO_4$ oder wässr. oder alkohol. HCl, wobei die drei letzteren Kondensationsmittel darstellen, die auch in den genannten Lösungsmittel verwandt werden können. Als Kondensationsmittel kommen sonst noch in Betracht :

Metall-, insbesondere Alkalialkoholate, Alkaliamide, Alkalihydride (NaH), starke Säuren wie Trifluoressigsäure, p-Toluolsulfonsäure, aber auch Dehydratisierungsmittel wie Dicyclohexylcarbodiimid. Bei den Halogenatomen, die bei dem Verfahren a) in Betracht kommen, handelt es sich vorzugsweise im Cl, Br oder auch die halogenähnliche Gruppe $N_3$. Falls X eine Alkoxygruppe, eine Alkylmercaptogruppe, eine Alkylaminogruppe oder eine Dialkylaminogruppe ist, handelt es sich im allgemeinen um solche mit Alkylresten mit 1 bis 6 C-Atomen. Bedeutet X eine Acyloxygruppe, so handelt es sich vorzugsweise um eine aliphatische Acylgruppe aus 2 bis 6 C-Atomen. Bei einer Aryloxygruppe handelt es sich bevorzugt um ein Phenoxygruppe.

Das Verfahren b) ist dadurch gekennzeichnet, daß man das aktivierte Malonsäurederivat unter Verwendung eines geeigneten inerten Lösungsmittels wie z. B. Benzol, Toluol, Xylol, Ether, THF, Dioxan oder DMF bei Raumtemperatur oder vorteilhafter bei der Siedetemperatur des jeweiligen Lösungsmittels umsetzt. In manchen Fällen erweist sich auch der Zusatz einer tertiären organischen Base wie z. B. Pyridin oder Triethylamin als günstig für den Ablauf der Reaktion. Die Umsetzung mit Malonsäure erfolgt am besten unter Verwendung von starken Säuren wie HCl, $H_2SO_4$, Trifluoressigsäure oder Polyphosphorsäure.

Das Verfahren c) ist dadurch gekennzeichnet, daß die Zyklisierung zum Siebenring unter den gleichen Bedingungen erfolgt, wie für das Verfahren (a) beschrieben.

Die für das Verfahren a), b) und c) verwendeten Ausgangsmaterialien können, soweit sie nicht bekannt sind, beispielsweise auf folgenden Wegen erhalten werden :

a) Eine Verbindung der allgemeinen Formel (V)

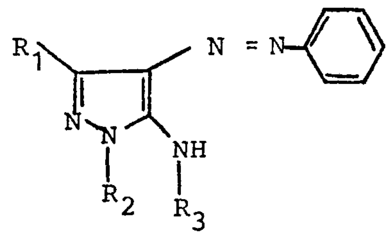

(V)

worin $R_1$, $R_2$ und $R_3$ (mit Ausnahme von Wasserstoff), die oben angegebenen Bedeutungen haben, wird mit einer Verbindung der allgemeinen Formel (VI)

$$Y—CO—CH_2—COX \qquad (VI)$$

wobei X die oben angegebenen Bedeutungen hat und Y ein Chlor- oder Bromatom, eine Azidogruppe ($N_3$) oder eine Alkyloxy- oder Aryloxygruppe bedeutet, in einem inerten Lösungsmittel wie Dioxan, THF, Chloroform, Benzol oder Toluol bei Temperaturen zwischen 0 und 200 °C mit oder ohne Zusatz eines Säureakzeptors, zu einer Verbindung der allgemeinen Formel (VIII)

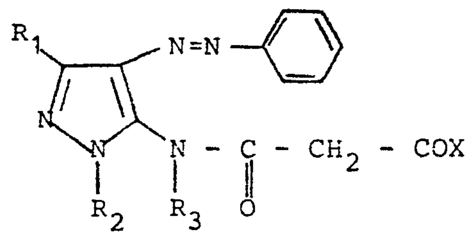

(VII)

umgesetzt.

Bei dieser Verbindung wird dann die Azogruppe reduktiv gespalten, wobei die Aminoverbindung der allgemeinen Formel II entsteht. Die reduktive Spaltung der Azogruppe kann erfolgen durch katalytische Hydrierung (mit Pd, Pt, Raney-Nickel in Alkoholen, Dioxan, THF bei 0-60 °C und bei 1-50 Atm. $H_2$-Druck) oder durch chemische Reduktion z. B. mit Natriumdithionit in wäßriger oder alkoholischer Lösung, mit $SnCl_2$ in HCl oder mit Zink in Eisessig oder neutraler, saurer oder alkalischer wäßriger Lösung.

Die Ausgangsmaterialien der allgemeinen Formel (V) sind auf dem von F. A. Amer, A. H. Harhash und M. L. Awad [Z. Naturforsch. *33 b,* 660-662 (1978)] beschriebenen Wege zugänglich oder lassen sich wie folgt synthetisieren :

Ein Pyrazolon der allgemeinen Formel (VIII)

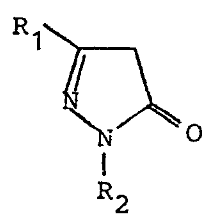

(VIII)

wird umgesetzt mit Benzoldiazoniumchlorid in Eisessig bei 0-5 °C zum Phenylhydrazon der allgemeinen Formel (IX)

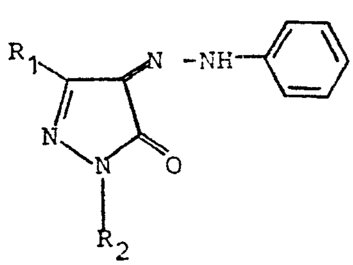

(IX)

das dann in siedendem $POCl_3$ in 5-Position chloriert wird zum 4-Benzolazo-5-chlorpyrazol der allgemeinen Formel (X)

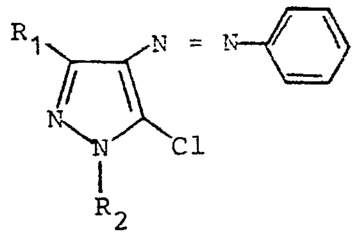

(X)

Anschließend wird der Chlorrest ausgetauscht gegen ein entsprechend substituiertes Amin $R_3NH_2$, wobei $R_3$ die oben angegebenen Bedeutungen mit Ausnahme von Wasserstoff hat, bei 100-160 °C mit oder ohne Lösungsmittel, wodurch die Verbindung der allgemeinen Formel (V) entsteht.

Verbindungen der allgemeinen Formel (II) können außerdem auf folgendem Weg erhalten werden : Eine Verbindung der allgemeinen Formel (X)

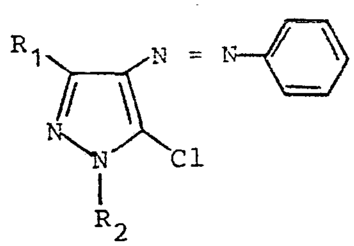

(X)

(Synthese siehe oben)
wird mit einer Verbindung der allgemeinen Formel (XI)

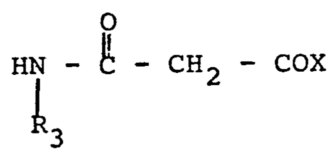

(XI)

wobei $R_3$ (mit Ausnahme von Wasserstoff) die angegebenen Bedeutungen hat und für X die angegebenen Reste mit Ausnahme von Halogen, $NH_3$, Alkylamino und Azido ($N_3$) in Frage kommen ; zu einer Verbindung der allgemeinen Formel (VII) umgesetzt. Man arbeitet dabei im allgemeinen in einem inerten Lösungsmittel wie z. B. Dioxan, THF, DMF unter Zusatz eines Protonenakzeptors wie NaH, Natriumamid oder feinverteiltem Natrium. Die Temperaturen der Reaktion liegen zwischen 0 und 150 °C.

Verbindungen der allgemeinen Formel (II), in denen X = Halogen ist, können z. B. aus Verbindungen der allgemeinen Formel (II), in denen X = OH ist, durch Umsetzung mit Halogenierungsmitteln wie Thionylchlorid oder Phosphorpentachlorid bei Temperaturen zwischen 0 bis 100 °C in inerten Lösungsmitteln wie Benzol, Toluol, Dioxan oder THF erhalten werden. Die Carbonsäuren der allgemeinen Formel (II) (X = OH) kann man beispielsweise auch aus dem entsprechendem Ester (X = Alkyloxy-, Aryloxy- oder Benzyloxy-) erhalten, indem dieser unter milden Bedingungen in die Säure überführt wird, z. B. durch Hydrierung (bei X = Benzyloxy) oder durch sehr milde Hydrolyse (bei X = Aryloxy- oder verzweigter Alkyloxyrest wie tert. Butoxy-). Verbindungen der allgemeinen Formel (II), in denen X eine Acyloxygruppe ist kann man auch aus den entsprechenden Halogeniden durch Umsetzung mit den entsprechenden Metallsalzen in inerten Lösungsmitteln wie Aceton, Dioxan, Ether bei Temperaturen zwischen − 20 und 100 °C erhalten.

Verbindungen der allgemeinen Formel (II), in denen X eine Azidogruppe ist, lassen sich beispielsweise aus den entsprechenden Halogeniden durch Umsetzung mit Alkaliaziden in inerten Lösungsmitteln wie Aceton, Dioxan, DMSO bei Temperaturen zwischen 0 und 100 °C erhalten, oder aus Estern (X = O-Alkyl bzw. O-Aryl) durch Umsetzung mit Hydrazin, gegebenfalls in einem inerten Lösungsmittel wie Ethanol, Dioxan, THF bei 0 bis 100 °C und anschließender Reaktion des Hydrazins mit salpetriger Säure oder nitrosen Gasen in inerten Lösungsmitteln wie Alkoholen, Dioxan, DMF bei Temperaturen zwischen 0 und 50 °C.

Die Ausgangsstoffe der allgemeinen Formel (XI) werden, soweit sie nicht bekannt sind, wie folgt erhalten :

Ein Amin $R_3—NH_2$, wobei $R_3$ (mit Ausnahme von Wasserstoff) die oben angegebenen Bedeutungen hat, wird mit einer Verbindung der allgemeinen Formel (VI), worin X die oben genannten Bedeutungen hat (ausgenommen Halogen, $NH_2$, Alkylamino und Azido bei X) und Y ein Chlor- oder Bromatom, eine Azidogruppe oder eine Alkyloxy- oder Aryloxygruppe bedeutet, in einem inerten Lösungsmittel wie

Dioxan, THF, Chloroform, Aceton oder auch in einem Überschuß der Verbindung VI bei Temperaturen zwischen 0 und 200 °C umgesetzt. Es kann hierbei beispielsweise analog Chem. Ber. *17*, 739 ff (1884) oder J. Indian Chem. Soc. *37*, 591-593 (1960) verfahren werden.

b) Bei einer Verbindung der allgemeinen Formel (V) wird die Azogruppe unter den oben angegebenen Bedingungen reduktiv gespalten zu einer Verbindung der allgemeinen Formel (III).

c) Verbindungen der allgemeinen Formel (IV) sind zugänglich durch Umsetzung von Verbindungen der allgemeinen Formel (III) mit Verbindungen der allgemeinen Formel (VI), wobei die Aminogruppe in 4-Stellung des Pyrazolringes selektiv abreagiert. Die Reaktion kann erfolgen in einem inerten Lösungsmittel wie z. B. Benzol, Toluol, Dioxan, THF, Chloroform mit oder ohne Zusatz eines Säureakzeptors zwischen 0 und 150 °C.

## Beispiel 1

1,3,8-Trimethyl-5,6,7,8-tetrahydropyrazolo(3,4-b) (1,5)diazepin-1H,4H-5,7-dion.

a) 4-Amino-1,3-dimethyl-5-methylaminopyrazol. 23 g (0,1 Mol) 4-Benzolazo-1,3-dimethyl-5-methylaminopyrazol werden in 250 ml Ethanol mit 60 g Raney-Nickel bei 60 °C und 50 Atmosphären Wasserstoffdruck hydriert. Nach Ende der Wasserstoffaufnahme wird vom Katalysator abgesaugt und die Reaktionslösung im Vakuum eingedampft. Der Rückstand wird mit Ether/Petrolether verrieben und der Niederschlag abgesaugt. Das Produkt ist rein genug für die weiteren Umsetzungen. Fp. : 87 °C.

b) 4-α-Ethoxycarbonylacetylamino-1,3-dimethyl-5-methylaminopyrazol

1,4 g (0,01 Mol) 4-Amino-1,3-dimethyl-5-methylaminopyrazol werden in 20 ml Toluol gelöst, und unter Eiskühlung wird langsam 1 ml Malonsäuremonomethylesterchlorid (0,012 Mol) zugetropft und eine Stunde bei Raumtemperatur nachgerührt. Das Toluol wird im Vakuum abgezogen, der Rückstand in Chloroform aufgenommen, mit eiskalter $NaHCO_3$-Lösung und Wasser gewaschen und mit $Na_2SO_4$ getrocknet. Nach Verdampfen des Lösungsmittels bleibt ein gelbliches Öl zurück, das nicht kristallisierte.

c) 1,3,8-Trimethyl-5,6,7,8-tetrahydropyrazolo(3,4-b) (1,5)diazepin-1H,4H-5,7-dion.

2,4 g 4-α-Ethoxycarbonylacetylamino-1,3-dimethyl-5-methyl-aminopyrazol (0,01 Mol), gelöst in 100 ml Ethanol, werden mit 15 ml einer 1-molaren Natriummethanolat-Lösung versetzt und 8 Stunden bei Raumtemperatur gerührt. Anschließend wird mit alkoholischer HCl neutralisiert, im Vakuum eingedampft, der Rückstand mit $CHCl_3$ aufgenommen, filtriert und erneut eingedampft. Nach Zugabe von Ether wird der Rückstand kristallin und kann abgesaugt werden. Umkristallisation erfolgt aus Isopropanol/Diisopropylether. Fp. : 202 °C.

## Beispiel 2

1-Ethyl-3,8-dimethyl-5,6,7,8-tetrahydropyrazolo-(3,4-b) (1,5)diazepin-1H,4H-5,7-dion.

a) 4-Benzolazo-1-ethyl-5-(N-methyl-N-α-methoxycarbonylacetylamino)-3-methylpyrazol.

1) Zu einer Lösung von 14,5 g (0,1 Mol) Methoxycarbonyl-N-methylacetamid (Darstellung siehe H. Ulrich *et al.*, J. Org. Chem. *27*, 2160-2162) in 100 ml trockenen DMF unter Stickstoffatmosphäre gibt man bei Raumtemperatur 2,4 g NaH (0,1 Mol) hinzu und rührt 30 Min. nach. Dann wird unter Kühlung eine Lösung von 24,8 g 4-Benzolazo-5-chlor-3-methyl-1-ethylpyrazol (0,1 Mol) in 50 ml DMF langsam zugetropft und eine Stunde auf 50 °C erwärmt. Man setzt 10 ml Ethanol und 5 ml Eisessig hinzu und dampft die Lösung im Vakuum ein. Der Rückstand wird in $CHCl_3$ aufgenommen, mit Wasser gewaschen, mit $Na_2SO_4$ getrocknet und eingedampft. Umkristallisation aus Petrolether liefert gelborangefarbene Kristalle vom Schmelzpunkt 52-53 °C.

2) 125 g 4-Benzolazo-1-ethyl-3-methyl-5-methylaminopyrazol (0,5 Mol) werden mit 61 ml Malonsäuremonomethylesterchlorid (0,55 Mol) in 250 ml Benzol umgesetzt, bis die HCl-Entwicklung beendet ist. Anschließend wird das Benzol im Vakuum abgezogen, der Rückstand in $CHCl_3$ aufgenommen, mit kalter $NaHCO_3$-Lösung und Wasser gewaschen, getrocknet und eingedampft. Umkristallisation aus Petrolether liefert Kristalle, identisch mit den unter 2 a) 1) beschriebenen Kristallen.

b) 4-Amino-1-ethyl-3-methyl-5-(N-methyl-N-α-methoxycarbonylacetylamino)-pyrazol.

3,4 g 4-Benzolazo-1-ethyl-5-(N-methyl-N-α-methoxycarbonylacetylamino)-3-methylpyrazol (0,01 Mol), gelöst in 100 ml Ethanol, werden mit 10 g Raney-Nickel bei 60 °C und 50 Atmosphären Wasserstoffdruck hydriert. Nach Ende der Wasserstoffaufnahme wird vom Katalysator abfiltriert und die Reaktionslösung im Vakuum eingedampft. Als Rückstand blieb ein farbloses Öl, das sich nicht kristallisieren ließ.

c) 1-Ethyl-3,8-dimethyl-5,6,7,8-tetrahydropyrazolo-(3,4-b) (1,5)diazepin-1H,4H-5,7-dion.

Obiges Öl wird in Ethanol gelöst, und nach Zugabe von 1 ml konz. HCl wird solange unter Rückfluß gekocht, bis das Ausgangsmaterial verschwunden ist. Anschließend wird die Lösung mit alkoholischer KOH-Lösung neutralisiert, im Vakuum eingedampft, der Rückstand wird in $CHCl_3$ aufgenommen, getrocknet und eingedampft. Das zurückbleibende Öl kristallisiert beim Verreiben mit Ether. Fp. : 160 °C.

Beispiel 3

1-Isopropyl-3,8-dimethyl-5,6,7,8-tetrahydropyrazolo(3,4-b) (1,5)diazepin-1H,4H-5,7-dion.

Unter Eiskühlung wird zu einer Lösung von 280 mg Kohlensuboxid [Darstellung siehe H. Staudinger, S. Bereza, Ber. *41*, 4461 (1908)] (0,04 Mol) in 70 ml Ether eine Lösung von 672 mg 4-Amino-1-isopropyl-3-methyl-5-methylaminopyrazol (0,004 Mol) langsam zugetropft. Anschließend wird noch eine Stunde bei 0 °C gerührt und der Niederschlag abgesaugt. Umkristallisation erfolgt aus Diisopropylether. Fp. : 163 °C.

Nach den beschriebenen Vorschriften wurden außerdem folgende Verbindungen hergestellt :

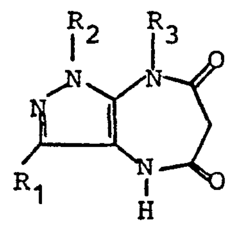

| Beispiel | $R_1$ | $R_2$ | $R_3$ | Fp(°C) |
|---|---|---|---|---|
| 4 | $CH_3$ | $C_2H_5$ | 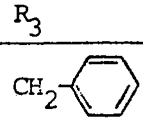 | 177 |
| 5 | $CH_3$ | $CH_3$ | $CH_3O-CH_2-CH_2-$ | 168 |
| 6 | $C_2H_5$ | $CH_3$ | $CH_3$ | 192 |
| 7 | 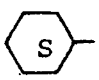 | $CH_3$ | $CH_3$ | 205 |
| 8 | 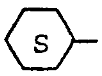 | $C_2H_5$ | $CH_3$ | 202 |
| 9 | $CH_3$ | H | $CH_3$ | 318 |
| 10 | $n-C_3H_7$ | $CH_3$ | $CH_3$ | 174 |
| 11 | $n-C_3H_7$ | $C_2H_5$ | $CH_3$ | 173 |
| 12 | $CH_3$ | 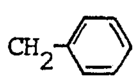 | $CH_3$ | 189 |
| 13 | $CH_3$ | $CH_3$ | 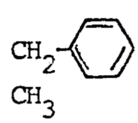 | 234 |
| 14 | H | $CH_3$ | $CH_3$ | 233 |
| 15 | $CH_3$ | $CH_3$ | $n-C_3H_7$ | 224 |
| 16 | $CH_3$ | $CH_3$ | 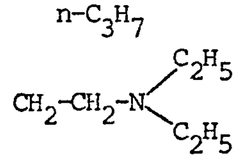 | 160 |
| 17 | $CH_3$ | $CH_3$ | $C_2H_5$ | 227 |

Tabelle (Fortsetzung)

| Beispiel | $R_1$ | $R_2$ | $R_3$ | Fp(°C) |
|---|---|---|---|---|
| 18 | $CH_3$ | $CH_3$ | 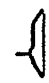 | 268 |
| 19 | $CF_3$ | $CH_3$ | $CH_3$ | 158 |
| 20 | $CH_3$ | 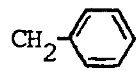 | 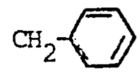 | 198 |

**Ansprüche** (für die Vertragsstaaten : BE, CH, DE, FR, GB, IT, LI, NL, SE)

1. Verbindungen der allgemeinen Formel (I)

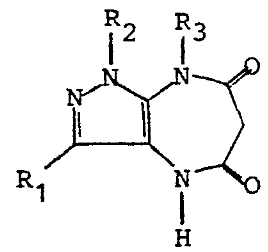

(I)

worin $R_1$ und $R_2$ gleich oder verschieden sind und Wasserstoffatome oder Alkylgruppen mit 1-6 C-Atomen darstellen, wobei einer der Reste $R_1$ und $R_2$ jeweils auch eine Benzyl-, Trifluormethyl oder Phenylgruppe sein kann, und $R_3$ ein Wasserstoffatom, eine Alkylgruppe mit 1-6 C-Atomen, die gegebenenfalls durch eine Phenylgruppe, eine Alkoxygruppe mit 1-6 C-Atomen, eine Trifluormethyl oder eine Dialkylaminogruppe mit 2-12 C-Atomen oder eine Cycloalkylgruppe mit 3-6 C-Atomen substituiert ist, eine Alkenyl- oder Alkinylgruppe mit 2-6 C-Atomen, eine Cycloalkylgruppe mit 3-6 C-Atomen oder eine Carbalkoxygruppe mit 2-6 C-Atomen sein kann.

2. Verbindungen gemäß Anspruch 1, worin $R_1$ Methyl, $R_2$ Wasserstoff, Methyl, Äthyl, Isopropyl oder Phenyl und $R_3$ Methyl bedeuten.

3. Verfahren zur Herstellung von Verbindungen gemäß Anspruch 1, dadurch gekennzeichnet, daß man

a) eine Verbindung der allgemeinen Formel (II)

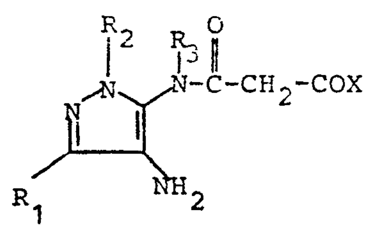

(II)

wobei $R_1$, $R_2$, $R_3$ (mit Ausnahme von Wasserstoff) die oben angegebenen Bedeutungen haben und X eine Hydroxygruppe, eine Mercaptogruppe, ein Halogenatom, eine Alkoxygruppe, eine Alkylmercaptogruppe, eine Aminogruppe, eine Alkylaminogruppe, eine Dialkylaminogruppe, eine Benzolyloxygruppe, eine Aryloxygruppe, eine Acyloxygruppe oder die Gruppe $N_3$ bedeutet, zum Siebenring zyklisiert ; oder

b) eine Verbindung der allgemeinen Formel (III)

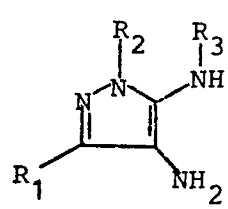

(III)

wobei $R_1$, $R_2$ und $R_3$ (mit Ausnahme von Wasserstoff) die oben angegebenen Bedeutungen haben, mit einem aktivierten Malonsäurederivat, z. B. Malonsäuredihalogenid, Malonester, Kohlensuboxyd, Meldrumsäure oder mit Malonsäure selbst zum Siebenring kondensiert ; oder

c) eine Verbindung der allgemeinen Formel (IV)

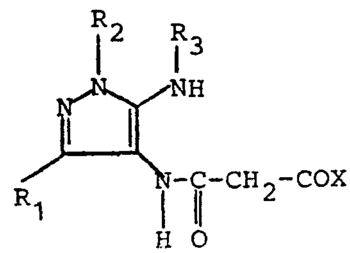

(IV)

wobei $R_1$, $R_2$, $R_3$ (mit Ausnahme von Wasserstoff) die oben angegebenen Bedeutungen haben und für X die unter a) angegebenen Gruppen in Frage kommen, mit den unter a) beschriebenen Verfahren zum Siebenring zyklisiert.

**Anspruch** (für den Vertragsstaat AT)

Verfahren zur Herstellung von Verbindungen der allgemeinen Formel (I)

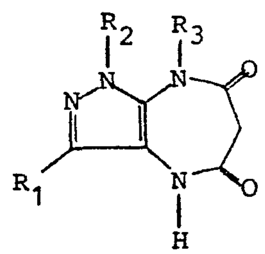

(I)

worin $R_1$ und $R_2$ gleich oder verschieden sind und Wasserstoffatome oder Alkylgruppen mit 1-6 C-Atomen darstellen, wobei einer der Reste $R_1$ und $R_2$ jeweils auch eine Benzyl-, Trifluormethyl oder Phenylgruppe sein kann, und $R_3$ ein Wasserstoffatom, eine Alkylgruppe mit 1-6 C-Atomen, die gegebenenfalls durch eine Phenylgruppe, eine Alkoxygruppe mit 1-6 C-Atomen, eine Trifluormethyl oder eine Dialkylaminogruppe mit 2-12 C-Atomen oder eine Cycloalkylgruppe mit 3-6 C-Atomen substituiert ist, eine Alkenyl- oder Alkinylgruppe mit 2-6 C-Atomen, eine Cycloalkylgruppe mit 3-6 C-Atomen oder eine Carbalkoxygruppe mit 2-6 C-Atomen sein kann, dadurch gekennzeichnet, daß man

a) eine Verbindung der allgemeinen Formel (II)

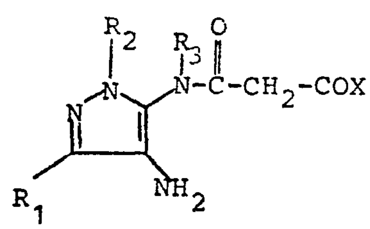

(II)

wobei $R_1$, $R_2$, $R_3$ (mit Ausnahme von Wasserstoff) die oben angegebenen Bedeutungen haben und X eine Hydroxygruppe, eine Mercaptogruppe, ein Halogenatom, eine Alkoxygruppe, eine Alkylmercaptogruppe, eine Aminogruppe, eine Alkylaminogruppe, eine Dialkylaminogruppe, eine Benzolyloxygruppe, eine Aryloxygruppe, eine Acyloxygruppe oder die Gruppe $N_3$ bedeutet, zum Siebenring zyklisiert ; oder

b) eine Verbindung der allgemeinen Formel (III)

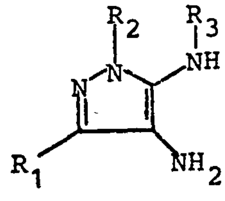

(III)

wobei $R_1$, $R_2$ und $R_3$ (mit Ausnahme von Wasserstoff) die oben angegebenen Bedeutungen haben, mit einem aktivierten Malonsäurederivat, z. B. Malonsäuredihalogenid, Malonester, Kohlensuboxyd, Meldrumsäure oder mit Malonsäure selbst zum Siebenring kondensiert ; oder

c) eine Verbindung der allgemeinen Formel (IV)

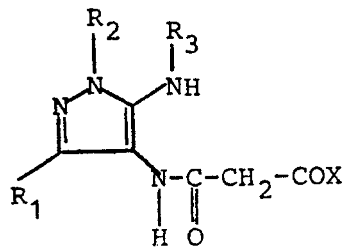

(IV)

wobei $R_1$, $R_2$, $R_3$ (mit Ausnahme von Wasserstoff) die oben angegebenen Bedeutungen haben und für X die unter a) angegebenen Gruppen in Frage kommen, mit den unter a) beschriebenen Verfahren zum Siebenring zyklisiert.

**Claims** (for the Contracting States : BE, CH, DE, FR, GB, IT, LI, NL, SE)

1. Compounds of the general formula (I)

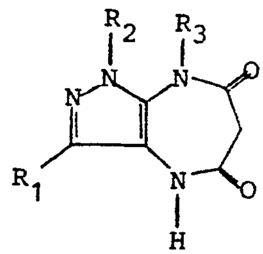

(I)

in which $R_1$ and $R_2$ are identical or different and represent hydrogen atoms or alkyl groups with 1-6 C atoms, it also being possible for one of the radicals $R_1$ and $R_2$ to be, in each case, a benzyl, trifluoromethyl or phenyl group, $R_3$ denotes a hydrogen atom, an alkyl group which has 1-6 C atoms and is optionally substituted by a phenyl group, an alkoxy group with 1-6 C atoms, a trifluoromethyl group, a dialkylamino group with 2-12 C atoms or a cycloalkyl group with 3-6 C atoms, an alkenyl or alkynyl group with 2-6 C atoms, a cycloalkyl group with 3-6 C atoms or a carbalkoxy group with 2-6 C atoms.

2. Compounds according to claim 1, wherein $R_1$ is $CH_3$, $R_2$ is hydrogen, $CH_3$, $C_2H_5$, i-$C_3H_7$ or phenyl, $R_3$ is $CH_3$.

3. Process for the preparation of compounds according to claim 1, which comprises

a) cyclizing a compound of the general formula (II)

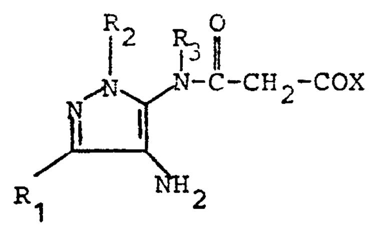

(II)

in which $R_1$, $R_2$, $R_3$ (with the exception of hydrogen) have the above-mentioned meanings and X denotes a hydroxyl group, a mercapto group, a halogen atom, an alkoxy group, an alkylmercapto group, an amino group, an alkylamino group, a dialkylamino group, a benzolyloxy group, an aryloxy group, an acyloxy group or the group $N_3$ to form the seven-membered ring ; or

b) subjecting a compound of the general formula (III)

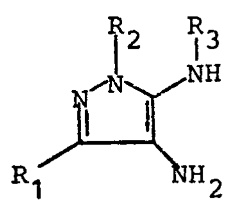

(III)

in which $R_1$, $R_2$ and $R_3$ (with the exception of hydrogen) have the above-mentioned meaning, to a condensation reaction with an activated malonic acid derivative, for example a malonic acid dihalide, a malonic acid ester, carbon suboxide or Meldrums's acid, or with malonic acid itself, to form the seven-membered ring ; or

c) cyclizing a compound of the general formula (IV)

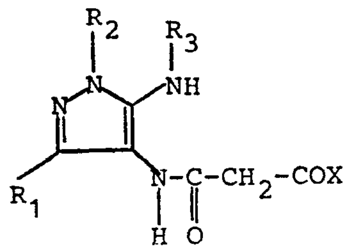

(IV)

in which $R_1$, $R_2$, $R_3$ (with the exception of hydrogen) have the above-mentioned meaning and X can be the groups indicated under a), by the processes described under a) to form the seven-membered ring.

**Claim** (for Contracting State AT)

Process for the preparation of compounds of the general formula (I)

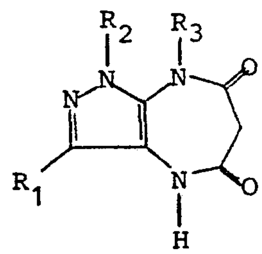

(I)

in which $R_1$ and $R_2$ are identical or different and represent hydrogen atoms or alkyl groups with 1-6 C atoms, it also being possible for one of the radicals $R_1$ and $R_2$ to be, in each case, a benzyl, trifluoromethyl or phenyl group, and $R_3$ denotes a hydrogen atom, an alkyl group which has 1-6 C atoms and is optionally substituted by a phenyl group, an alkoxy group with 1-6 C atoms, a trifluoromethyl group, a dialkylamino group with 2-12 C atoms or a cycloalkyl group with 3-6 C atoms, an alkenyl or alkynyl group with 2-6 C atoms, a cycloalkyl group with 3-6 C atoms or a carbalkoxy group with 2-6 C atoms which comprises

a) cyclizing a compound of the general formula (II)

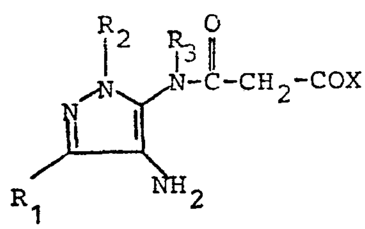

(II)

in which $R_1$, $R_2$, $R_3$ (with the exception of hydrogen) have the above-mentioned meanings and X denotes a hydroxyl group, a mercapto group, a halogen atom, an alkoxy group, an alkylmercapto group, an amino group, an alkylamino group, a dialkylamino group, a benzolyloxy group, an aryloxy group, an acyloxy group or the group $N_3$ to form the seven-membered ring ; or

b) subjecting a compound of the general formula (III)

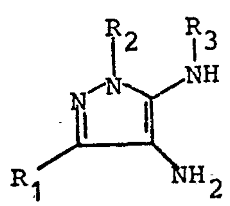

(III)

in which $R_1$, $R_2$ and $R_3$ (with the exception of hydrogen) have the above-mentioned meaning, to a condensation reaction with an activated malonic acid derivative, for example a malonic acid dihalide, a malonic acid ester, carbon suboxide or Meldrum's acid, or with malonic acid itself, to form the seven-membered ring ; or

c) cyclizing a compound of the general formula (IV)

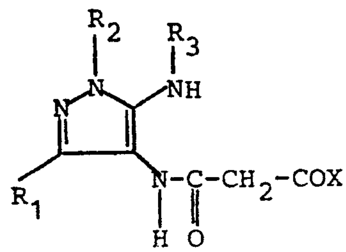

(IV)

in which $R_1$, $R_2$, $R_3$ (with the exception of hydrogen) have the above-mentioned meaning and X can be the groups indicated under a), by the processes described under a) to form the seven-membered ring.

**Revendications** (pour les Etats contractants : BE, CH, DE, FR, GB, IT, LI, NL, SE)

1. Composés de formule générale (I)

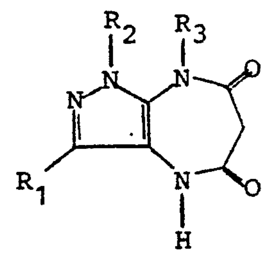

(I)

dans laquelle $R_1$ et $R_2$ sont identiques ou différents et représentent des atomes d'hydrogène ou des groupes alcoyle ayant de 1 à 6 atomes de carbone, l'un des radicaux $R_1$ et $R_2$ pouvant être également un groupe benzyle, trifluorométhyle ou phényle, et $R_3$ peut être un atome d'hydrogène ; un groupe alcoyle ayant de 1 à 6 atomes de carbone, qui peut être éventuellement substitué par un groupe phényle, un groupe alcoxy ayant de 1 à 6 atomes de carbone, un groupe trifluorométhyle ou un groupe dialcoylamino ayant de 2 à 12 atomes de carbone ou un groupe cycloalcoyle ayant de 3 à 6 atomes de carbone ; un groupe alcényle ou alcynyle ayant de 2 à 6 atomes de carbone, un groupe cycloalcoyle ayant de 3 à 6 atomes de carbone ou un groupe carbalcoxy ayant de 2 à 6 atomes de carbone.

2. Composés selon la revendication 1, dans lesquels $R_1$ représente un groupe méthyle, $R_2$ représente l'hydrogène, un groupe méthyle, éthyle, isopropyle ou phényle, et $R_3$ représente un groupe méthyle.

3. Procédé pour la préparation de composés selon la revendication 1, caractérisé en ce que

a) on cyclise un composé de formule générale (II)

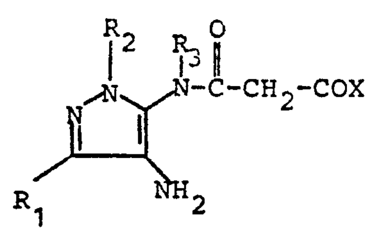

(II)

dans laquelle $R_1$, $R_2$, $R_3$ ont les significations indiquées ci-dessus (à l'exception de l'hydrogène) et X représente un groupe hydroxy, un groupe mercapto, un atome d'halogène, un groupe alcoxy, un groupe alcoylmercapto, un groupe amino, un groupe alcoylamino, un groupe dialcoylamino, un groupe benzoyloxy, un groupe aryloxy, un groupe acyloxy, ou le groupe $N_3$, en un noyau à 7 chaînons ; ou

b) on condense un composé de formule générale (III)

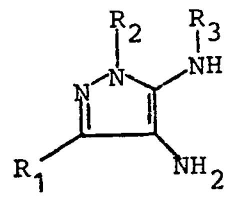

(III)

dans laquelle $R_1$, $R_2$ et $R_3$ ont les significations indiquées ci-dessus (à l'exception de l'hydrogène) avec un dérivé de l'acide malonique activé, par exemple un dihalogénure de l'acide malonique, un ester malonique, du sous-oxyde de carbone, l'acide de Meldrum ou avec l'acide malonique lui-même, en un noyau de 7 chaînons ; ou

c) on cyclise un composé de formule générale (IV)

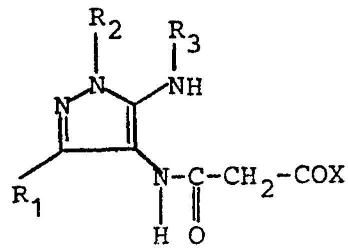

(IV)

dans laquelle $R_1$, $R_2$, $R_3$ ont les significations indiquées ci-dessus (à l'exception de l'hydrogène) et X représente les groupes indiqués en a), selon le procédé décrit en a), en un noyau à 7 chaînons.

**Revendication** (pour l'Etat contractant AT)

Procédé pour la préparation de composés de formule générale I

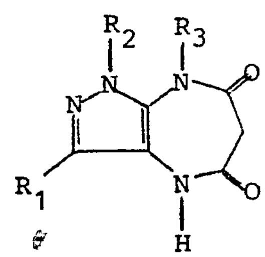

(I)

dans laquelle $R_1$ et $R_2$ sont identiques ou différents et représentent des atomes d'hydrogène ou des groupes alcoyle ayant de 1 à 6 atomes de carbone, l'un des radicaux $R_1$ et $R_2$ pouvant être également un groupe benzyle, trifluorométhyle ou phényle, et $R_3$ peut être un atome d'hydrogène ; un groupe alcoyle ayant de 1 à 6 atomes de carbone, qui peut être éventuellement substitué par un groupe phényle, un groupe alcoxy ayant de 1 à 6 atomes de carbone, un groupe trifluorométhyle ou un groupe dialcoylamino ayant de 2 à 12 atomes de carbone ou un groupe cycloalcoyle ayant de 3 à 6 atomes de carbone ; un groupe alcényle ou alcynyle ayant de 2 à 6 atomes de carbone, un groupe cycloalcoyle ayant de 3 à 6 atomes de carbone ou un groupe carbalcoxy ayant de 2 à 6 atomes de carbone, lequel procédé est caractérisé en ce que

a) on cyclise un composé de formule générale (II)

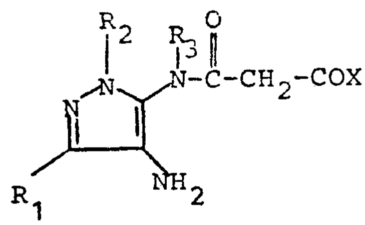

(II)

dans laquelle $R_1$, $R_2$, $R_3$ ont les significations indiquées ci-dessus (à l'exception de l'hydrogène) et X représente un groupe hydroxy, un groupe mercapto, un atome d'halogène, un groupe alcoxy, un groupe alcoylmercapto, un groupe amino, un groupe alcoylamino, un groupe dialcoylamino, un groupe

benzoyloxy, un groupe aryloxy, un groupe acyloxy ou le groupe $N_3$, en un noyau à 7 chaînons ; ou

b) on condense un composé de formule générale (III)

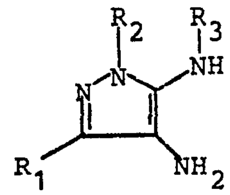

(III)

dans laquelle $R_1$, $R_2$ et $R_3$ ont les significations indiquées ci-dessus (à l'exception de l'hydrogène), avec un dérivé de l'acide malonique activé, par exemple un dihalogénure de l'acide malonique, un ester malonique, le sous-oxyde de carbone, l'acide de Meldrum ou avec l'acide malonique lui-même, en un noyau à 7 chaînons ; ou

c) on cyclise un composé de formule générale (IV)

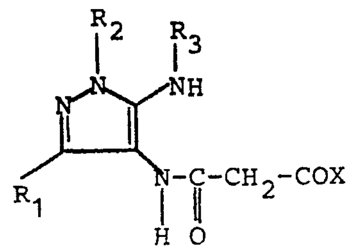

(IV)

dans laquelle $R_1$, $R_2$, $R_3$ ont les significations indiquées ci-dessus (à l'exception de l'hydrogène) et X représente les groupes indiqués en a), selon le procédé décrit en a), en un noyau à 7 chaînons.